# EUROPEAN PATENT SPECIFICATION

(11) **EP 3 244 921 B1**
(45) Date of publication and mention of the grant of the patent: **28.08.2019**
(21) Application number: 16705838.7
(22) Date of filing: 14.01.2016
(51) Int. Cl.: A61K 39/29, C12N 15/85, A61K 39/00

(54) **AN IMMUNOGENIC VACCINE AGAINST THE HCV AND/OR HBV**
IMMUNOGENER IMPFSTOFF GEGEN HCV UND/ODER HBV
VACCIN IMMUNOGÈNE CONTRE LE VHC ET/OU LE VHB

(30) Priority: 15.01.2015 PL 41095015
(43) Date of publication of application: 22.11.2017
(73) Proprietor: Uniwersytet Gdanski, 80-309 Gdansk (PL)
(72) Inventor: CZARNOTA, Anna, 81-017 Gdynia (PL); GRZYB, Katarzyna, 83-050 Bielkówko (PL)
(74) Representative: Pawlowska, Justyna
(86) International application number: PCT/PL2016/000002
(87) International publication number: WO 2016/114676

(56) References cited:
- WO-A1-02/10416
- WO-A1-2008/108918
- WO-A1-2009/061739
- VIETHEER PATRICIA T K ET AL: "Immunizations with chimeric hepatitis B virus-like particles to induce potential anti-hepatitis C virus neutralizing antibodies", ANTIVIRAL THERAPY- AN OFFICIAL PUBLICATION OF THE INTERNATIONAL SOCIETY FOR ANTIVIRAL RESEARCH, MTM PUBLICATIONS, LONDON, GB, vol. 12, no. 4, 1 January 2007 (2007-01-01), pages 477-487, XP009180161, ISSN: 1359-6535
- PION CORINNE ET AL: "Characterization and immunogenicity in mice of recombinant influenza haemagglutinins produced inLeishmania tarentolae", VACCINE, ELSEVIER LTD, GB, vol. 32, no. 43, 12 August 2014 (2014-08-12), pages 5570-5576, XP029062555, ISSN: 0264-410X, DOI: 10.1016/J.VACCINE.2014.07.092
- ANNA CZARNOTA ET AL: "Immunogenicity of Leishmania-derived hepatitis B small surface antigen particles exposing highly conserved E2 epitope of hepatitis C virus", MICROBIAL CELL FACTORIES, vol. 351, no. 27, 13 April 2016 (2016-04-13), page 2832, XP055270806, DOI: 10.1186/s12934-016-0460-4

## Description

The invention concerns an immunogenic vaccine designated for administration in prophylaxis of the HCV and/or HBV viral infections, and the method of obtaining it. The invention further concerns chimeric protein and a composition containing the protein.

About 170 million individuals in the world are infected with the Hepatitis C Virus (HCV), which represents about 2-3% of the global population. The highest infection rate is found in Egypt where the HCV-infected account for as much as 20% of the population. The tests conducted all over Poland reveal that the sub-population of individuals infected with the Hepatitis C Virus ranges between 1.9 and 4%. However, because no attempt has been taken to hold tests which would embrace the entire population and gauge the actual scale of the problem, the number of the infected is only estimated at about 700 thousand. The modes of transmitting HCV infection are varied. As early as at the outset of HCV research and investigations a note was made of the observed relationship between the symptoms of the disease and the patient's prior contact with blood or blood derivatives. Today, thanks to the introduced hospital procedures developed countries rarely record HCV infections during medical procedures. In the developing countries, however, they continue to rank among the most frequent modes of transmission. As for the developed countries, the most common modes of infection include intravenous injection drug use (about 60% of new infections), mother-to-child virus transmission, and sexual contacts. Considering the astounding variety of human behaviours which carry the risk of coming in contact with blood or blood derivative products, one can list many more activities potentially carrying the risk of HCV infection. Let us but name e.g. needle-involving cosmetic procedures such as piercing, tattooing, or acupuncture. Most of such procedures are performed in regions where no routine screening tests for HCV infection are held; hence it is difficult to gauge their impact on the spreading of the epidemic. About 80% of the HCV infections become chronic, where no specific symptoms may occur for years, and for this reason HCV infection is frequently referred to as 'silent epidemic'. 20% of chronic infections lead to severe liver damage, hepatic cirrhosis, and consequently the development of the hepatocellular carcinoma. Frequently, the last resort option left for the patients is liver transplantation, though this, unfortunately, does not guarantee the cure. The transplanted liver is immediately infected with the virus particles circulating in the blood or nested in cells other than those of the liver. Within the time ranging between several and several dozen days after the transplantation the patients are recorded to succumb to increased viremia. For years, the standard anti-viral treatment consisted in a combination therapy with Interferon and Ribavirin. Unfortunately, the therapy carried severe side effects and its effectiveness did not exceed 50%. Intense research effort of the recent years has broadened our knowledge of the biology of the HCV and in effect has led to the discovery of medicines acting directly on the virus particles (direct acting antivirals - DAAs). Available today, are drugs acting on both the viral proteases such as Telaprevir or Boceprevir, and on the RNA polymerase, e.g. Sofosbuvir; the effectiveness of the latter reaches as high as 90%. Unfortunately, the prices of the drugs, just as the cost of hospitalising the infected, are very high and impose a substantial burden on both the patients and the state. Considering all of the above aspects, development of an effective and commonly available vaccine which would protect against the infection is a major goal of the current HCV research.

HCV is a relatively small enveloped virus of the *Flaviviridae* family. Its genetic material is single-stranded RNA of positive polarity. The virus genome is associated with the multimeric protein core. The envelope forming the outer part of the virus is a lipid membrane with the protective E1 and E2 glycoproteins, forming the noncovalent E1/E2 heterodimer, anchored therein. Both proteins are strongly glycosylated and play a major role in the virus life cycle. They are involved in the process of the virus particle's entering into the host cell and in the process of depositing the infectious virus particles. Because of its RNA genetic material and no proof-reading activity of the RNA polymerase, the HCV demonstrates substantial genetic variability, which is the key factor enabling the virus to avoid the immune response. According to the most recent phylogenetic research, one can recognise seven genotypes and numerous subtypes of the virus, their variability reaching as high as 35%. Moreover, when infecting the organism, the virus diversifies forming closely related variants within a single organism infected. Furthermore, the E2 protein sequence has the so-called hypervariable regions (HVR) which play the key role in the infection process. The virus's high genetic variability makes it extremely difficult to design new drugs, vaccines in particular. To make things worse, the virus has other strategies at hand to evade the host immune system (immune evasion), e.g. it impedes the intracellular signalling pathways responsible for the production of interferons, hinders activation of the antigen-presenting cells and the cell response, and moves directly from cell to cell without being exposed to contact with the host immune system. In addition, the glycans on the particle surface enable the virus to mask its immunogenic regions (the glycan shield). The above strategies make designing an effective HCV vaccine an extremely tough challenge.

The ideal prophylactic vaccine should be geared against the strongly conserved epitopes accessible on the virus surface, which would induce both strong cell response from the T lymphocytes, and humoral response from the neutralising antibodies defending against the antigens of the E1E2 glycoproteins of the HCV. Through the steric effect or through binding to the site involved in the interaction between the virus particle and the receptor of the host cell, the antibodies prevent virus penetration into the cell and the spreading of the infection. One of the epitopes for the neutralising antibodies is the strongly conserved QLINTNGSWHIN region located in position 412 to 423 of the E2 envelope glycoprotein. The region is recognisable to the broadly neutralising antibodies, capable to bind to the E2 glycoprotein of most of the HCV genotypes. The same site also plays the key role in the binding of the virus to the CD81 cell receptor which participates in viral penetration into the host cell. Moreover, the epitope is linear, which means that in order to enhance its antiviral immunogenicity it can be used as the peptide antigen of the surface carriers, i.e. virus-like particles proposed in this invention.

Virus-like particles (VLPs) are small, highly immunogenic structures formed of multimers of the viral proteins. Thanks to their multimeric, spatial structure, virus-like particles trigger aggregation of the BCRs (B cell receptors) on the B surface of the lymphocytes, and in effect activate humoral response of the organism. Furthermore, they are capable of stimulating response from T lymphocytes, specifically from the helper, cytotoxic, and dendritic cells. In view of their strong immunogenic properties, they are used in new generations of vaccines, e.g. in the vaccine against the Human Papilloma Virus (HPV) available in Poland since 2007, or the vaccine against the Hepatitis B Virus (HBV).

The genetic material of the Hepatitis B Virus is formed of a partially double-strand DNA which codes the virus's structural and functional proteins in partially overlapping reading frames. The HBV virion is ca. 42 nm in diameter, and contains the C protein core. The core is shielded in a protein-lipid envelope formed by the surface antigen of the HBV virus - HbsAg; the latter occurs in three forms: long, medium, and short. The short form of the protein - sHBsAg, where the amino acids are ca. 226 long, demonstrates interesting properties of creating virus-like particles. Those small structures, ca. 22 nm in diameter, are not virulent, but highly immunogenic and capable of triggering strong immune response. The tertiary structure of the sHBsAg forms hydrophilic loops. On one of the loops, there is a strongly conserved determinant 'a' stimulating the strongest immune response. Due to its ability to form strongly immunogenic VLP particles, and its strongly conserved and exposed determinant 'a', the sHBsAg protein is used in the available anti-HBV vaccines. The vaccines are designed under the DNA recombination technique and produced in the yeast expression system. The system allows for obtaining the correctly folded sHBsAg protein in large volumes at low cost. The protein can be expressed in many other systems, mammalian included, where, unlike in the yeast system, it is glycosylated correctly and secreted to the medium. Unfortunately, protein expression in mammalian cells involves high production costs.

Due to its strong immunogenicity and capability of triggering strong cell and humoral responses, determinant 'a' of the HBsAg protein is frequently employed as the potential carrier of the human pathogen antigens. The research of recent years has studied the potential vaccines based on the sHBsAg protein which carries the antigens of e.g. the polio virus, human immunodeficiency virus (HIV), malaria parasite, and chikungunya virus. Furthermore, the description of invention EA 005313 B discloses a method of obtaining multivalent antigens by chemical combination of peptides into the so-called dendritic structures, following which the structures are chemically linked to the epitope carriers, e.g. the HBsAg protein.

The description of another invention, US 8.551.484 A, discloses neutralising antibodies of a broad spectrum of action, which interact with region 412 - 423 of the E2 glycoprotein of the HCV.

In the study below, with the view of obtaining a bivalent vaccine against the HBV and HCV chimeric protein was designed, where region 412-425 of the E2 envelope glycoprotein of HCV was inserted in determinant 'a' of the HBsAg protein originating from HBV, subtype adw2. Expression of the chimeric protein was obtained in a system based on a parasitic protozoa, the *Leishmania tarentolae. L. tarentolae* is a unicellular, eukaryotic organism infectious to geckos but demonstrating no pathogenicity to humans. The protein expression system based on *L. tarentolae* allows for obtaining high expression of correctly folded proteins with the N-glycosylation pattern, similar to that of the mammalian cells, though with the costs kept low, the handling easy, and posing no difficulty with increasing the culture scale, comparable to the ease of the yeast or bacterial cultures.

The purpose of the invention is to form virus-like particles and in effect create a bivalent vaccine against the HBV and HCV.

As such, the invention offers e.g. the following several advantages:
- triggering the immune response in the form of antibodies against HBV and/or HCV
- obtaining a bivalent vaccine designated for use in prophylaxis and/or treatment of HBV and HCV, which when administered protects from and/or suppresses the morbidity caused by HBV and/or HCV
- attaining the potential application of the chimeric protein in the form of vaccine for the purposes of prevention/prophylaxis and/or treatment of the HBV and/or HCV infections.

The invention's purpose is to provide a method for obtaining the vaccine. The method consisting the following step:
- obtaining biologically active and immunogenic virus-like particles composed of the small envelope protein of the HBV subtype adw2 (HBsAg), characterised by:
   - high immunogenicity
   - properties triggering humoral and cellular immune responses
- incorporating region 412-425 of the E2 glycoprotein of the HCV into determinant 'a', characterised by:
   - low variability among the HCV genotypes
   - presence of a binding site for the virus neutralising antibodies
   - linear nature
- attaining the correct exposition of the E2 412-425 region on the surface of the HBsAg protein and its accessibility to the neutralising antibodies,
- obtaining virus-like particles for the first time in the *Leishmania tarentolae* system the advantages of which are the following:
   - correct protein folding
   - presence of post translational modifications (e.g. N-glycosylation, where the glycosylation pattern is similar to that of the mammalian pattern)
   - high overexpression of proteins
   - low cost of the culture and its easy handling
   - possible suspension culture, which enables problem-free increase of the production scale
- ensuring easy process of purifying the HBsAg_412-425 particles, consisting in single-stage gradient ultracentrifugation

The invention concerns a vaccine containing determinant 'a' of the HBsAg protein of the HBV, subtype adw2, and region 412-425 of the E2 envelope glycoprotein of the HCV.The encoding sequence of determinant 'a' is as follows:

5'-TGCACAACTCCTGCTCAAGGAACCTCTATGTTTCCCT

CATGTTGCTGTACAAAACCTACGGACGGAAACTGCAC-3'

The sequence of region 412-425 of the glycoprotein is encoded by the following sequence:
5'-CAACTGATCAACACCAACGGCAGTTGGCACATCAATAGCACG-3'

Preferably, the vaccine is chimeric protein.The isolated chimeric protein, in the form of the vaccine defined above, for use in vaccination - for administration against viruses HBV and/or HCV.

The chimeric protein defined above for use in prophylaxis of the HBV and/or HCV viral infections.

The method of obtaining the vaccine defined above, consisting of the following stages:
- insertion of the below-given sequence of region 412-425 of the E2 envelope glycoprotein of the HCV in the position corresponding to determinant 'a' of the HBsAg protein of the HBV virus, subtype adw2,
   5'-CAACTGATCAACACCAACGGCAGTTGGCACATCAATAGCACG-3'
- cloning of the synthesised fragment into plasmid pLEXSY_I-blecherry3 using restriction sites BglII and Notl under the control of promoter T7 with tetracycline operator,
- the use of the prepared plasmid in electroporation of the *Leishmania tarentolae* cells,
- expression of protein HBsAg_412-425 in the *Leishmania tarentolae* cells, cell lysis, and particle formation,
- purification of the virus-like particles in single-stage gradient ultracentrifugation.

The composition for use in prophylaxis of HCV and/or HBV infections in a patient, where the composition contains the chimeric protein defined above, in a pharmaceutically acceptable carrier.

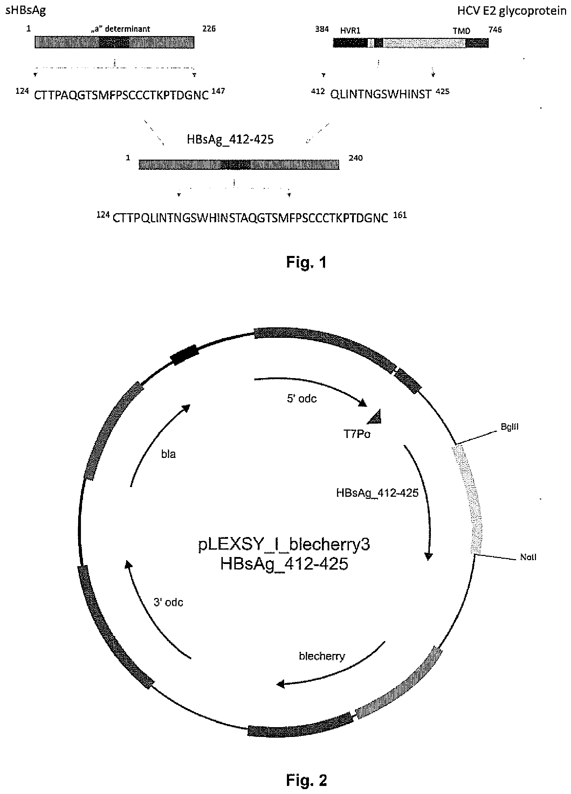

The expression vector being plasmid pLEXSY_I_blecherry3 containing the nucleotide sequence of the chimeric gene HBsAg_412-425 and restriction sites BglII, NotI, presented on Fig.2.

The following terms are defined for the purposes of the present invention:
**HCV** - denotes Hepatitis C Virus, Hepatitis C.

**HBV** - denotes Hepatitis B Virus, Hepatitis B.

**antigen** - denotes any substance which stimulates the organism's immune system to produce antibodies geared against the antigen.

**fusion protein** - is the same as chimeric protein.

**bivalent vaccine** - denotes the vaccine which triggers immune response against two different pathogens (or two different pathogen serotypes). In this particular case, it denotes the vaccine which triggers immune response against both the HBV and/or the HCV viruses.

**chimeric proteins** - denote the proteins produced in effect of expression of recombinant genes obtained in combination of two or more genes, each coding different proteins originally. In this particular case, they denote the combination of the short form of the HBV virus surface antigen (HBsAg) and region 412-425 of the E2 glycoprotein of the HCV, where the E2 412-425 region is inside determinant 'a' of the HBsAg.

**determinant 'a'** - denotes the highly immunogenic region of the small envelope protein HBsAg, located in position 124-147 of the small envelope protein of the HBV virus - sHBsAg (Genbank accession number - AF397207.1).

**expression** - denotes production of proteins in effect of translation of the protein coding genes.

**hypervariable region** - denotes the regions in the amino acid sequence of glycoprotein E2, which demonstrate increased variability. Variability is the key factor enabling the HCV particle to evade the immune response.

**cellular response** - denotes the specific immune response of the organism, involving the occurrence of T lymphocytes with receptors on their surface. The receptor's binding to a specific antigen triggers direct, cytotoxic response from the T lymphocyte, or activation of other effector cells.

**humoral response** - denotes the specific immune response of the organism, involving the production of antibodies by the activated B lymphocytes.

**epitope, antigen determinant** - denotes the site on the antigen which specifically binds the antibody/immunoglobulin (e.g. the E2 412-423 region specifically binding the AP33 antibody). Among the epitopes one can distinguish linear epitopes - where the region binding the antibody is linear and does not depend on the protein tertiary structure, and the denaturation factors do not affect the ability of binding the antibody to the epitope, and conformational epitopes - where the binding of the antibody depends on epitope presentation in the protein tertiary structure.

**virus neutralisation** - denotes the ability of the neutralising antibodies to bind to the virus particle in such a way that the latter is unable to penetrate the host cell.

**glycosylation pattern** - denotes the pattern of the hydrocarbon particles covalently bound to a protein particle.

**region 412-425** - denotes the region in position 412 to 425 of the E2 glycoprotein of the HCV, isolate H77 (Genbank accession number - AF011751) of the QLINTNGSWHINST amino acid sequence. The region contains a strongly conserved linear epitope recognised by the neutralising antibodies of a broad spectrum of action, are able to bind to the E2 glycoprotein originating from most HCV genotypes. In this particular case, region 412-425 was used to form chimeric protein HBsAg_412-425, where the region is inside determinant 'a' of the HBsAg protein.

**peptide 412-425** - denotes the synthesised peptide of the *QLINTNGSWHINST* amino acid sequence.

**antibody AP33** - denotes the mouse monoclonal antibody of the QLINTNGSWHIN sequence, binding to the linear epitope of the E2 glycoprotein of the HCV. The antibody demonstrates properties neutralising the HCV. (A.Owsianka,A.W. Tarr,V. S. Juttla et al., "Monoclonal antibody AP33 defines a broadly neutralizing epitope on the hepatitis C virus E2 envelope glycoprotein," Journal of Virology, vol. 79, no.17, pp. 11095-11104, 2005).

**HBV virus, subtype adw** - denotes the variant of the HBV found to have determinants 'a', 'd', and 'w'. The HBV virus has three determinants, out of which determinant 'a' is universal and common for all subtypes. The flanking regions of determinant 'a' are called determinants d/y and w/r. Thanks to the type of the amino acid in position 122 for determinant d/y, and in position 160 for determinant w/r, each isolate of the HBV virus can be ascribed to a specific subtype.

**patient** - denotes a person uninfected with HBV and/or HCV, who is vaccinated with chimeric protein HBsAg_412-425 to prevent HBV and/or HCV infection.

**infection prophylaxis** - denotes prevention of new HBV and/or HCV infections by administering prophylactic vaccinations of the individuals exposed to the HBV and/or HCV infections with chimeric protein HBsAg_412-425. The vaccination triggers permanent immune response in the form of memory cells which, if in contact with the HBV and/or HCV viruses, are able to neutralise and eliminate the HBV and/or HCV viruses from the organism of the vaccinated person.

### Description of the figures:

Fig. 1 - presents a diagram of the chimeric HBsAg_412-425 construct used in the study below. Region 412-425 originating from the E2 envelope glycoprotein of the HCV was inserted in position P127/A128 of the small envelope protein of the HBV virus (HBsAg), within determinant 'a'.
**Fig. 2** **-** presents a diagram of the plasmid pLEXSY_I-blecherry3 with the fragment coding protein HBsAg_412-425 cloned in under the control of the tetracycline promoter.

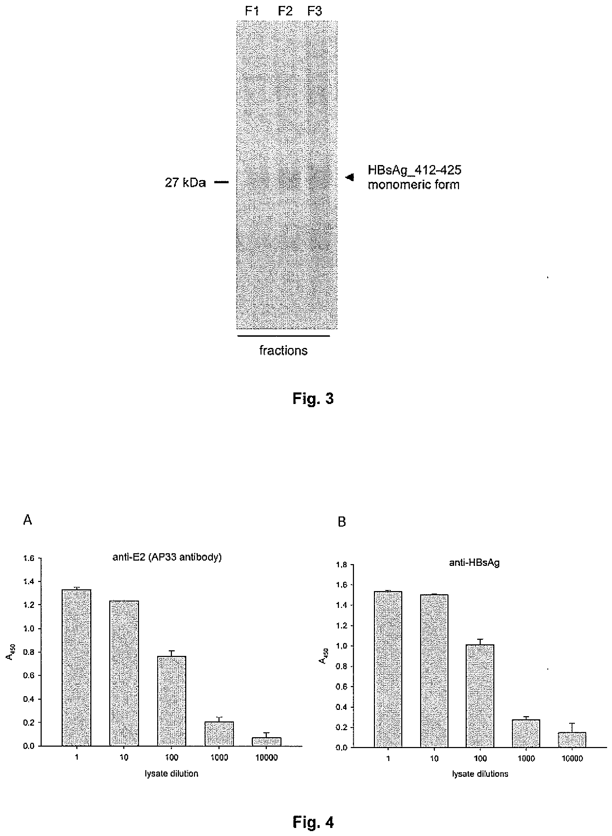

**Fig. 3** **-** presents the fractions containing protein HBsAg_412-425 upon ultracentrifugation, separated on SDS-PAGE gel under reducing conditions after their staining with Coomassie Blue. The arrow on the figure points to the monomeric form of protein HBsAg_412-425 with the molecular weight of t -27 kDa. The kDa molecular weight marker is positioned left of the gel.
**Fig. 4** **-** presents the result of the ELISA test, confirming a positive response from the antibodies to the chimeric HBsAg_412-425 particles. The plates were coated with L. *tarentolae* lysate containing chimeric protein HBsAg_412-425 in dilutions of the following ranges: 1, 1:10, 1:100, 1:1000, 1:10000. Subsequently, incubation with the AP33 antibodies (Fig. 4A) and anti-HBsAg (Fig. 4B) was conducted. The error bars mark the standard deviation.

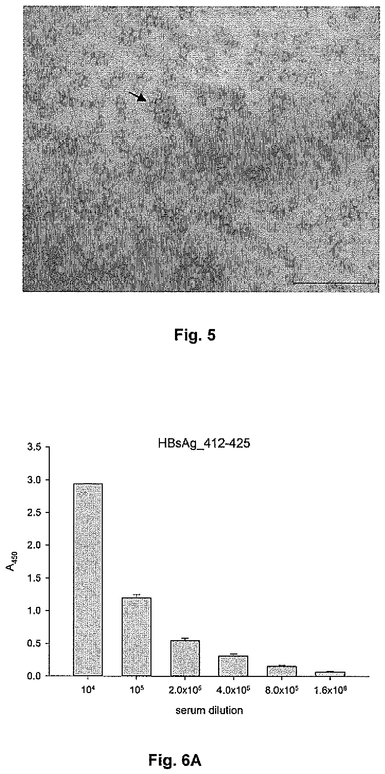

**Fig. 5** **-** presents a photograph of the preparation of the virus-like HBsAg_421-425 particles viewed under an electron microscope. In the experiment, the technique of negative staining with uranyl acetate was employed. The black arrow points to a single virus-like particle composed of chimeric protein HBsAg_412-425.

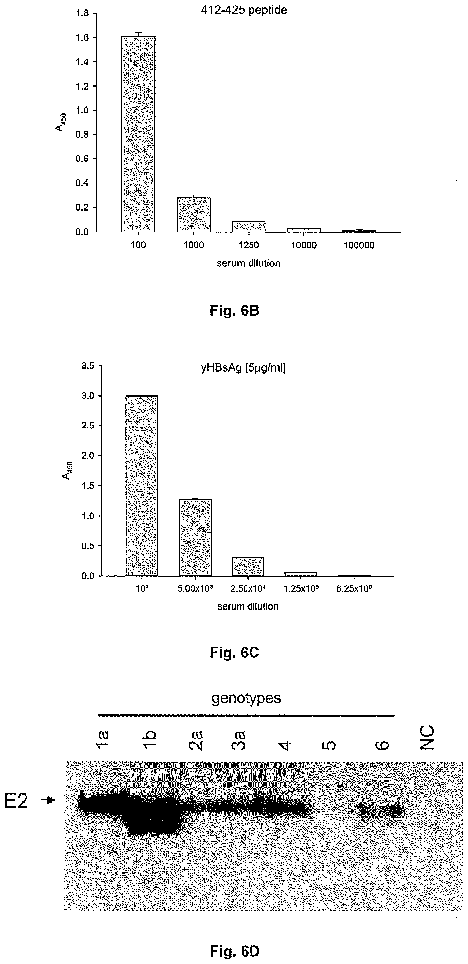

**Fig. 6 - A -** presents titre determination of the antibodies in the serum of mice immunised with protein HBsAg_412-425. 10 µg/ml of purified chimeric virus-like particles was applied to a 96 well plate. The protein was detected with sera in the following dilutions: 10⁴, 10⁵, 2x10⁵, 4x10⁵, 8x10⁵, 1.6x10⁶. **B** - 20 µg/ml of the earlier synthesised peptide 412-425 was applied to a 96 well plate. The peptide was detected with sera in the following dilutions: 10², 10³, 1.25x10³, 10⁴, 10⁵. **C -** presents interaction of the immune sera with yeast-derived HBsAg proteins. The ELISA plates were coated with 5 µg/mL of purified HBsAg protein from *P. pastoris* (yHBsAg). The protein was detected with sera in the following dilutions: 10³, 5x10³, 2.5x10⁴, 1.25x10⁵, 6.25x10⁵. Shown is one of two representative experiments in duplicate. The mean A₄₅₀ values and the standard deviations are shown on the *y*-axis. **D** - analysis of cross-reactivity of the HBsAg_412-425 sera to the E1E2 complex from different HCV genotypes. The figure represents western blotting in reducing conditions with HBsAg_412-425 sera diluted 1:500. As the antigen, extracts of the HEK293 cells transfected with plasmids expressing the E1E2 glycoproteins from different HCV genotypes were used. Non-transfected HEK293 cell lysate was used as the negative control (NC).

The invention is illustrated with the following exemplary, though not exhaustive, embodiments:

### EXAMPLE 1

### Obtaining expression of protein HBsAg_412-425 in the cells of the Leishmania tarentolae protozoa, and purifying the virus-like particles in gradient ultracentrifugation.

### Expression

The gene coding protein HBsAg of the HBV virus was synthesised (Life Technologies Inc. USA) based on a database sequence (GenBank accesion number - AF397207.1), the sequence for region 412-425 - 5'-CAACTGATCAACACCAACGGCAGTTGGCACATCAATAGCACG-3' was inserted in the position corresponding to determinant 'a' (Fig. 1). Then, the synthesised fragment was cloned into plasmid pLEXSY_I-blecherry3 (Jena Bioscience, Germany) using restriction sites Bglll and Notl, under the control of promoter T7 with tetracycline operator. The correctness of the insert introduction was verified in sequencing. The thus prepared expression vector (Fig. 2) was used in the electroporation of the *L*. *tarentolae* cells. The electroporation, polyclonal selection, cell culture, and induction of the expression of the chimeric protein were carried out in accordance with the procedure for the Lexsy system (Jena Bioscience, Germany).

### Lysis and formation of virus-like particles

The induced cultures (OD600∼4.5) were centrifuged (4500 rpm, 10 min). The cell pellets obtained from 100 ml of the culture were washed with the PBS buffer and then lysed in 10 ml of the buffer (0.6% Triton X-100, 0.6 mM reduced glutathione in PBS) and sonified. Then, the whole lysate was centrifuged (8500 rpm. 30 min.). After centrifugation, the supernatant was collected and incubated in room temperature for (12-48h).

### Purification

In order to purify the HBsAg_412-425 particles, OpitPrep gradient (Sigma) was prepared in PBS with fractions ranging from 6% to 30%, onto which the obtained cell lysate was placed in 5 ml portions. The samples were centrifuged (28000 rpm, 4°C, 16h). All fractions formed on the gradient were tested for the presence of protein HBsAg_412-425 with the western blot method using the AP33 antibodies. The purity of the protein was tested by polyacrylamide gel electrophoresis, SDS-PAGE (Fig. 3). On the Coomassie Blue stained gel, one can see the main strip corresponding to the weight of the monomeric form of protein HBsAg_412-425 (27 kDa) plus 3 additional strips of higher weights, most likely corresponding to multimeric forms of protein HBsAg_412-425. The fractions containing the desired protein in largest quantities were collected and densified using Amicon 30 NMLW centrifugal filters (Millipore Inc. USA). The Bradford method was employed to determine protein concentration (A=595 nm). Estimate calculations revealed that the protein quantities stood at about 20 mg/l of the culture. The thus purified preparation was stored at 4°C or at -70°C with admixture of glycerol (5-20%).

The above procedures enabled obtaining of the chimeric protein and effective single-stage purification of the virus-like particles composed of fusion protein HBsAg_412-425.

### EXAMPLE 2

### The ELISA test conducted and electron microscope photographs taken to verify the presence of the virus-like particles.

### ELISA

In order to perform the ELISA test, a 96-well plate was coated with cell lysate in PBS dilutions of 1 to 1:10000, and blocked with 5% milk in PBS-T (0.05% Tween20 in PBS). Then, applied were primary antibodies anti-HBsAg (1:1000) in PBST (Fig. 4B), or AP33 antibodies (1:1500) in PBST (Fig. 4A). In the experiment, HRP conjugated secondary antibodies were used at the dilution of 1:2000. The response was developed using the TMB substrate, and the result was read at the wavelength of 450 nm. The positive response of the antibodies to the chimeric HBsAg_412-425 particles confirms the correct exposure of region 412-425 on the surface of the HBsAg protein and its accessibility to the antibodies produced in contact with the antigen.

### Electron microscopy

In order to verify the presence of the virus-like particles, 10 µl of the sample was applied to nickel grids coated with a carbon film with Formvar admixture and stained with uranyl acetate. The preparations were viewed in an electron microscope (Fig. 5). The findings of the electron microscopy verified the presence of virus-like particles ∼ 22 nm in diameter, the fact corresponding with the literature data.

### EXAMPLE 3

### Immunisation of mice to verify immunogenicity of the virus-like particles, and determination of the titre of the antibodies active against the HBsAg_412-425 antigen in the obtained sera

In order to test the immunogenicity of the virus-like particles, the mouse model was employed in vaccinations. The purified HBsAg_412-425 antigen was suspended in PBS and mixed with the adjuvant (Addavax; Invivogen, USA) in the 1:1 proportion directly before the vaccination. A group of six BALB/c mice was inoculated subcutaneously with the antigen/adjuvant mixture at the dose of 15 µg/mouse. The control group of two BALB/c mice was inoculated with a PBS/adjuvant mixture. On the 14^{th} and 28^{th} day after the first inoculation, the mice were administered the antigen in a lower quantity, i.e. at the dose of 10 µg/mouse, whereas the control group was administered the PBS/adjuvant mixture. On the 42^{nd} day, all mice were bled and the collected serum subject to determination of the titre of the antibodies active against the whole antigen HBsAg_412-425 and region 412-425 in addition. The titre was determined under the ELISA method (Fig. 6A and B). The above experiment confirmed the presence of antibodies active against protein HBsAg_412-425, the titre of which was about 8x10⁵; it also confirmed the presence of antibodies active against region 412-425, the titre of which was about 1.25x10³. In both cases the negative result was assumed to represent values (n<0.15), where 0.15 is three times the value of the negative control (A₄₅₀=0.05). The immunisation results confirm a strong immune response in the form of antibodies active against the antigen given the form of chimeric protein HBsAg_412-425, including region 412-425 specific for the HCV. Additionally, detailed serum characterisation revealed cross-reactivity with purified yeast-derived HBsAg protein (yHBsAg) (Fig. 6C). The result indicated that the sera contained high titres of anti-HBsAg antibodies, suggesting that 14 aa foreign epitope insertion within a sHBsAg particle does not interfere dramatically with the humoral response against the sHBsAg protein itself. Furthermore, the tested sera were also able to cross-react with the E1E2 complexes from different HCV genotypes. As expected, the sera recognised the E2 glycoproteins from HCV genotypes 1a, 1b, 2b, 3a, 4, and 6, but failed to recognise the E2 derived from genotype 5 (Fig. 6D). That was due to the fact that the isolate had 4 amino acid changes in region 412-423 unlike the sequence used to create the HBsAg_412-425 protein. Considering the above, the 412-425_HBsAg sera showed broad cross-reactivity across various HCV genotypes.

### Literature:

1. Hindman S.J. i wsp. 2014: Historical epidemiology of hepatitis C virus (HCV) in selected countries. J. Viral Hepat. Suppl 1:5-33.
2. Frank C., Mohamed M.K., Strickland G.T., Lavanchy D., Arthur R.R., Magder L.S., El Khoby T., Abdel-Wahab Y., Aly Ohn E.S., Anwar W., Sallam I. 2000: The role of parenteral antischistosomal therapy in the spread of hepatitis C virus in Egypt. Lancet. 355: 887-891.
3. Halota W., Pawtowska M. 2002: Hepatitis C virus infection--a major problem in hepatology. Przegl Epid. 56. supl. 4:46-50.
4. 2003: Stanowisko grupy ekspertów w dziedzinie chorób zakaźnych dotyczqce leczenia wirusowego zapalenia wqtroby typu C. Medical Science Monitor. vol 9. supl. 6.
5. Garfein R. S., Vlahov D., Galai N. Doherty M. C., Nelson K. E. 1996: Viral infections in short-term injection drug users: the prevalence of the hepatitis C, hepatitis B, human immunodeficiency, and human T-lymphotropic viruses. Am J Public Health. v.86(5).
6. Stramer S. L., Glynn S. A., Kleinman S. H., Strong D. M., Caglioti S., Wright D. J., Dodd R. Y., Busch M.P. 2004: Detection of HIV-1 and HCV infections among antibody-negative blood donors by nucleic acid amplification testing. N Engl J Med. 351(8):760-8.
7. Alter M.J. 2007: Epidemiology of hepatitis C virus infection. World J Gastroenterol. 13(17): 2436-2441.
8. Kimberly A. Powers, Ruy M. Ribeiro, Keyur Patel, Stephen Pianko, Lisa Nyberg, Paul Pockros, Andrew J. Conrad, John McHutchison, Alan S. Perelson 2006: Kinetics of Hepatitis C Virus Reinfection After Liver Transplantation. Liver Transplantation, 12:207-216.
9. Ghany M. G., Nelson D. R., Strader D. B., Thomas D. L. Seeff L. B. 2011: Practice Guideline by the American Association for the Study of Liver Diseases.
10. Mangia A., Marcellin P., Kwo P. i wsp. 2014: All oral fixed-dose combination sofosbuvir/ledipasvir with or without ribavirin for 12 or 24 weeks in treatment-naive genotype 1 HCV-infected patients: the phase 3 ION-1 study. 49th European Association for the Study of the Liver International Liver Congress London, April 9-13, 2014. Abstract
11. Dubuisson J. 2007: Hepatitis C virus proteins. World J Gastroenterol. 13(17): 2406-2415.
12. Zein N. 2000: Clinical Significance of Hepatitis C Virus Genotypes. Clin Microbiol Rev. 13(2): 223-235.
13. Kelly P. Burke, Andrea L. Cox 2010: Hepatitis C Virus Evasion of Adaptive Immune Responses- A Model for Viral Persistence. Immunol Res. 47(1-3): 216-227.
14. Helle F., Duverlie G., Dubuisson J. 2011: The Hepatitis C Virus Glycan Shield and Evasion of the Humoral Immune Response. Viruses. 3(10), 1909-1932.
15. Ania Owsianka, Alexander W. Tarr, Vicky S. Juttla, Dimitri Lavillette, Birke Bartosch, Francois-Loic Cosset, Jonathan K. Ball, Arvind H. Patel 2005: Monoclonal Antibody AP33 Defines a Broadly Neutralizing Epitope on the Hepatitis C Virus E2 Envelope Glycoprotein. Journal of Virology. 11095-11104.
16. Teresa J. Broering, Kerry A. Garrity, Naomi K. Boatright, Susan E. Sloan, Frantisek Sandor, William D. Thomas, Jr., Gyongyi Szabo, Robert W. Finberg, Donna M. Ambrosino, Gregory J. Babcock 2009: Identification and Characterization of Broadly Neutralizing Human Monoclonal Antibodies Directed against the E2 Envelope Glycoprotein of Hepatitis C Virus. Journal of Virology, 12473-12482.
17. Jane A. Potter, Ania M. Owsianka, Nathan Jeffery, David J. Matthews, Zhen-Yong Keck, Patrick Lau, Steven K. H.Foung, Garry L. Taylor and Arvind H. Patel 2012: Toward a Hepatitis C Vaccine: The Structural Basis of Hepatitis C Virus Neutralization by AP33, a Broadly Neutralizing Antibody. J. Virol., 86(23):12923.
18. Natasha Kushnir, Stephen J. Streatfield, Vidadi Yusibov 2012: Virus-like particles as a highly efficient vaccine platform: Diversity of targets and production systems and advances in clinical development. Vaccine, 31 58-83.
19. Stephen Locarnini, Fabien Zoulim 2010: Molecular genetics of HBV infection. Antiviral Therapy, 15 Suppl 3:3-14.
20. RTS,S Clinical Trials Partnership 2011: First Results of Phase 3 Trial of RTS,S/AS01 Malaria Vaccine in African Children. New England Journal of Medicine, 367 (24) 2284-95.
21. VRC 311 Study Team 2014: Safety and tolerability of chikungunya virus-like particle vaccine in healthy adults: a phase 1 dose-escalation trial. The Lancet.
22. Netter H. J., MacNaughton T. B., Wai-Ping Woo, Tindle R., Gowans E. J. 2000: Antigenicity and Immunogenicity of Novel Chimeric Hepatitis B Surface Antigen Particles with Exposed Hepatitis C Virus Epitopes. Journal of Virology, 2130-2141.
23. Wan-Shoo Cheong, Heidi Edelgard Drummer, Hans-Jurgen Netter 2009: Delivery of a foreign epitope by sharing amino acid residues with the carrier matrix. Journal of Virological Methods, 158 35-40.
24. Romuald Patient, Christophe Hourioux, Pascal Vaudin, Jean-Christophe Pages, Philippe Roingeard 2009: Chimeric hepatitis B and C viruses envelope proteins can form subviral particles: implications for the design of new vaccine strategies. New Biotechnology, 25 (4).
25. Elodie Beaumont, Romuald Patient, Christophe Hourioux, Isabelle Dimier-Poisson, Philippe Roingeard 2013: Chimeric Hepatitis B Virus/Hepatitis C Virus Envelope Proteins Elicit Broadly Neutralizing Antibodies and Constitute a Potential Bivalent Prophylactic Vaccine. Hepatology, 57:1303-1313.
26. Breitling R., Klingner S., Callewaert N., Pietrucha R., Geyer A., Ehrlich G., Hartung R., Muller A., Contreras R., Beverley S. M. i wsp. 2002: Non-pathogenic trypanosomatid protozoa as a platform for protein research and production. Protein Expr Purif., 25(2):209-218.

## Claims

1. A vaccine consisting of:
a) determinant 'a' of the HBsAg protein of the HBV virus, subtype adw2, encoded by the following sequence: 
5'-TGCACAACTCCTGCTCAAGGAACCTCTATGTTTCCCT
CATGTTGCTGTACAAAACCTACGGACGGAAACTGCAC-3',
b) and region 412-425 of the E2 envelope glycoprotein of the HCV encoded by the following sequence: 5'-CAACTGATCAACACCAACGGCAGTTGGCACATCAATAGCACG-3'.

2. The vaccine according to claim 1, **characterised in that** the vaccine is chimeric protein.

3. The vaccine as defined in the claims 1-2 for use in vaccination against viruses HBV and/or HCV.

4. The protein for the use according to claim 3 for use in prophylaxis of the HBV and/or HCV viral infections.

5. A method of obtaining the vaccine defined in the claims 1-2, consisting of the following stages:
- insertion of the below-given encoding sequence of region 412-425 of the E2 envelope glycoprotein of the HCV in the position corresponding to determinant 'a' of the HBsAg protein of the HBV, subtype adw2
5'-CAACTGATCAACACCAACGGCAGTTGGCACATCAATAGCACG-3'
- cloning of the synthesised fragment into plasmid pLEXSY_I-blecherry3 using restriction sites BglII and NotI under the control of promoter T7 with tetracycline operator,
- the use of the prepared plasmid in electroporation of the *Leishmania tarentolae* cells,
- expression of protein HBsAg_412-425 in the *Leishmania tarentolae* cells, cell lysis, and particle formation,
- purification of the virus-like particles in single-stage gradient ultracentrifugation.

6. A composition for use in prophylaxis of HCV and/or HBV infections in a patient, **characterised in that** the composition contains the chimeric protein defined in the claims 1-2, in a pharmaceutically acceptable carrier.

7. An expression vector **characterised in that** it is plasmid pLEXSY_I_blecherry3 containing the nucleotide sequence of the chimeric gene HBsAg_412-425 and restriction sites BglII, NotI, presented on Fig.2.

## Patentansprüche

1. Ein Impfstoff bestehend aus den:
a) Determinante "a" des HBsAg Eiweißstoffes von dem HBV Virus, Subtyp adw2 codiert durch folgende Sequenz: 
5'-TGCACAACTCCTGCTCAAGGAACCTCTATGTTTCCCT
CATGTTGCTGTACAAAACCTACGGACGGAAACTGCAC-3',
b) und 412-425 Region der E2 Glykoproteine der HCV Virushülle codiert durch folgende Sequenz:
5'-CAACTGATCAACACCAACGGCAGTTGGCACATCAATAGCACG-3'.

2. Der Impfstoff nach Anspruch 1 **dadurch gekennzeichnet, dass** er ein Fusionseiweiß ist.

3. Der Impfstoff nach Anspruch 1-2 zur Anwendung bei den Impfungen gegen HBV und/oder HCV Virus.

4. Der Impfstoff zur Anwendung gemäß Anspruch 3 zur Anwendung bei Prophylaxe der HBV und/oder HCV viralen Infektionen .

5. Verfahren zur Impfstoffgewinnung von Anspruch 1-2 bestehend aus den folgenden Schritten:
- insertion der unten genannten Sequenz, die die 412-425 Region der E2 Glykoproteine der HCV Virushülle auf der Position codiert, die mit der Determinante "a" des HBsAg Eiweißes vom HBV Virus mit einem adw2 Subtyp, 5'-CAACTGATCAACACCAACGGCAGTTGGCACATCAATAGCACG-3' korrespondiert,
- klonierung des synthetisierten Teils in plEXSY_I-blecherry3 Plasmid unter Verwendung von Restriktionsstellen BglII und NotI, die durch T7 Promotor mit dem Tetrazyklin Operateur kontrolliert werden,
- anwendung des vorbereiteten Plasmid in der Elektroporation von *Leishmania tarentolae* Zellen,
- expression des HBsAg_412-425 Eiweißes in *Leishmania tarentolae* Zellen, Lyse der Zelle und Formation von Molekülen,
- säuberung virusähnlicher Molekülen während des einstufigen Ultrazentrifugierens im Dichtegradient.

6. Zusammensetzung zur Anwendung bei Prophylaxe der HCV und/oder HBV viralen Infektionen bei Patienten, **dadurch gekennzeichnet** der umfasst des Fusionsproteins von Anspruch 1-2 in pharmazeutisch annehmbarem Träger.

7. Expressionsvektor ist **dadurch gekennzeichnet, dass** er ein pLEXSY_I_blecherry3 Plasmid ist und umfasst das Nukleotidsequenz des HBsAg_412-425 Fusionsgens und BglII i Notl Restriktionsstellen enthält in 2 Abbildung dargestellt.

## Revendications

1. Le vaccin constitué des :
a) détérminant "a" de la protéine HBsAg du virus HBV - sous-type adw2, est codé par la séquence suivante: 
5'-TGCACAACTCCTGCTCAAGGAACCTCTATGTTTCCCT
CATGTTGCTGTACAAAACCTACGGACGGAAACTGCAC-3'
b) et la région 412-425 de la glycoprotéine E2 de l'enveloppe du virus HCV est codé par la séquence suivante:
5'-CAACTGATCAACACCAACGGCAGTTGGCACATCAATAGCACG-3'.

2. Le vaccin, selon la revendication 1" est **caractérisée en ce qu'**il est une protéine de fusion.

3. Le vaccin défini dans les revendication 1 et 2, pour utilisation dansla vaccination contre le virus HBV et/ou HCV.

4. Le vaccin pour utilisation selon la revendication 3 pour utilisation dans la prévention des infections virales HCV et/ou HBV .

5. Le procédé de la préparation du vaccin, défini dans les revendications 1 et 2 constitué des phases suivantes:
- insertion de la séquence mentionnée ci-dessous de codification de la région 412-425 de la glycoprotéine E2 de l'enveloppe du virus HCV dans une position correspondante au déterminant "a" de la protéine HBsAg du virus HBV - sous-type adw2
5'-CAACTGATCAACACCAACGGCAGTTGGCACATCAATAGCACG-3',
- clonage du fragment synthétisé au plasmide pLEXSY_I-blecherry3 en utilisant les fragments de restriction BglII et NotI contrôlés par le promoteur T7 avec opérateur tétracycline,
- utilisation du plasmide préparé dans l'électroporation des cellules *Leishmania tarentolae,*
- expression de la protéine HBsAg_412-425 dans les cellules *Leishmania tarentolae,* lyse cellulaire et formation des molécules,
- nettoyage des perticules pseudo-virales au cours d'une ultracentrigugation à un stage dans le gradient de densité.

6. La composition pour utilisation dans la prévention des infections virales HCV et/ou HBV chez les patients, **caractérisée** en ce qui comprend en protéine de fusion défini dans les revendications 1 et 2 et un véhicule pharmaceutiquement acceptable.

7. Le vecteur d'expression est **caractérisée en ce que** c'est un plasmide pLEXSY_I_blecherry3 qui comprend une séquence nucléotide du gène de fusion HBsAg_412-425 et des fragments de restriction BglII i NotI, présenté sur le figure 2.
